# EUROPEAN PATENT SPECIFICATION

(11) **EP 3 668 604 B1**
(45) Date of publication and mention of the grant of the patent: **13.10.2021**
(21) Application number: 18740229.2
(22) Date of filing: 16.07.2018
(51) Int. Cl.: A61Q 11/00, A61K 8/19, A61K 8/24, A61K 8/365, A61K 8/20

(54) **ORAL CARE COMPOSITION**
MUNDPFLEGEZUSAMMENSETZUNG
COMPOSITION DE SOINS BUCCO-DENTAIRES

(30) Priority: 18.08.2017 WO PCT/CN2017/098034; 18.09.2017 EP 17191668
(43) Date of publication of application: 24.06.2020
(73) Proprietor: Unilever Global IP Limited, Wirral, CH62 AZD (GB); Unilever IP Holdings B.V., 3013 AL Rotterdam (NL)
(72) Inventor: LI, Xiaoke, Shanghai, Linkong Economic Development Zone 200335 (CN); WANG, Jinfang, Shanghai Linkong Economic Development Zone 200335 (CN); XING, Huaiyong, Shanghai Pudong New District 200120 (CN)
(74) Representative: Tansley, Sally Elizabeth
(86) International application number: PCT/EP2018/069253
(87) International publication number: WO 2019/034348

(56) References cited:
- WO-A1-99/13852
- WO-A2-2010/115037
- US-A1- 2014 314 694

## Description

### Technical Field of the Invention

The present invention relates to oral care compositions such as tooth pastes, powders, gums, mouthwashes and the like. In particular the present invention relates to an oral care composition comprising bioactive glasses that results in reducing tooth sensitivity and/or tooth remineralization. This invention also relates to the use of such compositions for treating tooth hypersensitivity and/or remineralizing and/or whitening of teeth of an individual.

### Background of the Invention

Tooth hypersensitivity is a temporary induced pain sensation that affects up to 20% of the adult population. Hypersensitive teeth may be sensitive to temperature, pressure or chemical action.

The dentin of the tooth generally contains channels, called tubules, which provide for an osmotic flow between the inner pulp region of the tooth and the outer root surfaces. Tooth hypersensitivity may be related to the general increase in exposed root surfaces of teeth as a result of periodontal disease, toothbrush abrasion, or cyclic loading fatigue of the thin enamel near the dentin-enamel junction. When root surfaces are exposed, dentinal tubules are also exposed.

The currently accepted theory for tooth hypersensitivity is the hydrodynamic theory, based on the belief that open exposed dentinal tubules allow fluid flow through the tubules. This flow excites the nerve endings in the dental pulp. Clinical replica of sensitive teeth viewed in a SEM (scanning electron microscopy) reveal varying numbers of open or partially occluded dentinal tubules.

There are different approaches to treat tooth hypersensitivity. One approach is to reduce the excitability of the nerve in a sensitive tooth by using "nerve-depolarising agents" comprising strontium ions, potassium salts such as potassium nitrate, potassium bicarbonate, potassium chloride and the like. These nerve-depolarising agents function by interfering with neural transduction of the pain stimulus to make the nerve less sensitive. Another approach is to use "tubule blocking agents" that fully or partially occlude tubules such as polystyrene beads, apatite, polyacrylic acid, mineral hectorite clay and the like. These tubule blocking agents function by physically blocking the exposed ends of the dentinal tubules, thereby reducing dentinal fluid movement and reducing the irritation associated with the shear stress described by the hydrodynamic theory.

Bioactive and biocompatible glasses have been developed as bone replacement materials. Studies have shown that these glasses will induce or aid osteogenesis in a physiologic systems. Bioactive glasses, alone or incorporated into oral care compositions, are known to remineralize teeth. The bioactive glass incorporated into these oral care compositions releases remineralizing ions onto the tooth's surfaces and helps occlude the open tubules, reducing tooth sensitivity, and rebuilding the enamel layer.

The present inventors have now found unexpectedly that an oral care composition comprising bioactive glass, a water slightly soluble and/or soluble calcium source and a phosphate source is more efficient for treating tooth hypersensitivity. The oral care composition exhibited excellent tubule blockage efficacy to reduce tooth sensitivity. In addition, such composition also enhances the tooth remineralization efficacy and/or the deposition of benefit agents on tooth surfaces to further benefit teeth of an individual.

### Additional Information

US 6,338,751 B1 discloses a novel silica based bioactive glass composition that can be used in conjunction with a delivery agent such as a toothpaste, gel saliva, etc. having a particle size range<90 µm which will form a rapid and continuous reaction with body fluid due to the immediate and long term ionic release of Ca and P from the core silica particles, to produce a stable crystalline hydroxyl carbonate apatite layer deposited onto and into the dentin tubules for the immediate and long term reduction of dentin hypersensitivity.

US 6,365,132 discloses methods for whitening teeth including contacting teeth with an efficient amount of particulate bioactive glass are disclosed.

US 6,190,643 discloses a method for reducing the viability of detrimental oral microorganisms and for the whitening of teeth by treatment with bioactive glass.

WO 2010/041073 discloses a film comprising a bioactive glass and a water soluble polymer and a method of using such a film for tooth remineralization.

US2014/314694 discloses compositions comprising bioactive glass, a phosphate source and calcium phosphate in a carrier.

The additional information above does not describe an oral care composition comprising a bioactive glass, a water slightly soluble and/or soluble calcium source and a phosphate source, wherein the bioactive glass and the water slightly soluble and/or soluble calcium source are present in a weight ratio from 1:3 to 20:1, and wherein the calcium source is calcium chloride, calcium nitrate, calcium gluconate, calcium glycerophosphate or mixtures thereof, and especially such an oral care composition has excellent tubule blockage efficacy and is more efficient for treating tooth hypersensitivity.

### Tests and Definitions

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purpose of the present invention means a paste or gel dentifrice for use with a toothbrush. Especially preferred are tooth pastes suitable for cleaning teeth by brushing for about two minutes.

### Particle Size

"Particle size" for the purpose of the present invention means D50 particle size. The D50 particle size of a particulate material is the particle size diameter at which 50 wt% of the particles are larger in diameter and 50 wt% are smaller in diameter.

### Composite Particle

"Composite particle" for the purpose of the present invention means a particle comprising a first component core and a second component coating wherein the core and coating are composed of different materials.

### Refractive Index

Refractive index is quoted at a temperature of 25°C and a wavelength of 589 nm.

### pH

pH is quoted at atmospheric pressure and a temperature of 25°C. When referring to the pH of an oral care composition, this means the pH measured when 5 parts by weight of the composition is uniformly dispersed and/or dissolved in 20 parts by weight pure water at 25°C. In particular the pH may be measured by manually mixing 5 g oral care composition with 20 mL water for 30 s, then immediately testing the pH with indicator or a pH meter.

### Solubility

"Soluble" and "insoluble" for the purpose of the present invention means the solubility of a source (e.g., like calcium salts) in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Slightly soluble", therefore, is defined to mean a source that dissolves in water to give a solution with a concentration of greater than 0.001 moles per litre and less than 0.1 moles per litre.

### Substantially Free

"Substantially free of" for the purpose of the present invention means less than 3.0%, and preferably less than 2.0%, and more preferably less than 1.0% and most preferably less than 0.5% by weight, based on total weight of the oral care composition, including all ranges subsumed therein.

### Dual-Phase

"Dual-Phase" for the purpose of the present invention means a composition having two independent phases which are physically separate.

### Anhydrous Composition

"Anhydrous composition" for the purpose of the present invention means the water content of the composition is less than 3.0%, and preferably less than 2.0%, and more preferably less than 1.0% and most preferably less than 0.5% by total weight of the oral care composition.

### Viscosity

Viscosity of a tooth paste is the value taken at room temperature (25 °C) with a Brookfield Viscometer, Spindle No.4 and at a speed of 5 rpm. Values are quoted in centipoises (cP=mPa.s) unless otherwise specified.

### Remineralization

"Remineralization" for the purpose of the present invention means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Summary of the Invention

In a first aspect, the present invention is directed to an oral care composition comprising:
a) a bioactive glass;
b) a water slightly soluble and/or soluble calcium source;
c) a phosphate source; and
d) a physiologically acceptable carrier;

wherein the bioactive glass and the water slightly soluble and/or soluble calcium source are present in a weight ratio from 1:3 to 20:1; and
wherein the calcium source is calcium chloride, calcium nitrate, calcium gluconate, calcium glycerophosphate or mixtures thereof.

In a second aspect, the present invention is directed to a packaged oral care product comprising the oral care composition of the first aspect of this invention.

In a third aspect, the present invention is directed to a method for reducing sensitivity and/or remineralization and/or whitening of teeth of an individual comprising the step of applying the oral care composition of any embodiment of the first aspect to at least one surface of the teeth of the individual.

All other aspects of the present invention will more readily become apparent upon considering the detailed description and examples which follow.

### Detailed Description

Now it has been found that an oral care composition comprising a bioactive glass, a water slightly soluble and/or soluble calcium source, and a phosphate source is more efficient for treating tooth hypersensitivity. The oral care composition exhibited excellent tubule blockage efficacy to reduce tooth sensitivity. In addition, such composition also enhances the tooth remineralization efficacy and/or the deposition of benefit agents on tooth surfaces to further benefit teeth of an individual.

Bioactive glasses are a group of surface reactive glass-ceramic biomaterials comprising SiO₂, CaO, P₂O₅, Na₂O and small amounts of other oxides which exhibit bioactivity. The bioactivity of theses glasses is the result of complex reactions which take place on the surface of the glasses under physiological conditions, and which result in the formation of hydroxyl carbonated apatite on the surface of the glasses. A bioactive glass typically has the basis formula CaO-P₂O₅-Na₂O-SiO₂. Bioactive glasses may be made by melt processes or by sol-gel processing (see, for example, Hench, J.Am.Ceram.Soc.,81,7,1705-28,(1998) and Hench, Biomaterials, 19(1998),1419-1423).

The bioactive glass suitable for use in this invention is limited only to the extent that the same may be used in the mouth. Suitable bioactive glasses are described, for example in WO 2010/041073 (BIOFILM LTD), WO 2009158564 (NOVAMIN TECHNOLOGY INC), WO 99/13852 (UNIV MARYLAND), WO 2005/063185 (NOVAMIN TECHNOLOGY INC), WO 96/10985 (BIOXID OY), WO 97/27148 (UNIV MARYLAND), US 9168272 B2 (QUEEN MARY AND WESTFIELD COLLEGE), and/or US 2016/0051457 (QUEEN MARY AND WESTFIELD COLLEGE) all of which international applications are hereby incorporated by reference in their entirety.

Typically, the bioactive glass comprises from 40 to 96% by weight of silicon dioxide (SiO₂), from 0 to 35% by weight of sodium oxide (Na₂O), from 4 to 46% by weight of calcium oxide (CaO), and from 1 to 15% by weight of phosphorous oxide (P₂O₅). More preferably, the bioactive glass comprises from 40 to 60% by weight of silicon dioxide (SiO₂), from 5 to 30% by weight of sodium oxide (Na₂O), from 10 to 35% by weight of calcium oxide (CaO), and from 1 to 12% by weight of phosphorous oxide (P₂O₅). The bioactive glass may further comprise one or more elements selected from K, Ca, Mg, B, Sr, Ti, Al, N, Ag or F.

The bioactive glass may further comprise antimicrobial metal ions including but not limited to silver, copper and zinc ions. Preferably, these salts are included in a range from 0 to 15% by weight.

The most preferred bioactive glass is the original bioactive glass which was FDA approved and termed Bioglass®, which is also known as 45S5. The Bioglass® 45S5 comprises 45% by weight of silicon dioxide, 24.5% by weight of sodium oxide, 24.5% by weight of calcium oxide and 6% by weight of phosphorus oxide. The Bioglass® 45S5 suitable for use in this invention is commercially available, for example from Kunshan Chinese Technology New Materials Co., LTD.

The bioactive glass may be particulate as this allows for maximum surface area for contact with dental tissue. Thus preferably the composition comprises particles comprising the bioactive glass. Preferably from 10 to 100%, and especially, from 25 to 100%, and most especially, from 70% to 100% by weight of the particles comprising bioactive glass used in this invention have a D50 particle size from 100 nm to 50 microns, preferably from 500 nm to 30 microns, more preferably from 700nm to 20 microns, most preferably from 1 micron to 15 microns.

Typically, the oral care composition of the present invention comprises from 0.1 to 80% by weight of the bioactive glass, more preferably from 0.2 to 50%, most preferably from 1 to 30% based on the total weight of the oral care composition and including all ranges subsumed therein.

The water slightly soluble and/or soluble calcium source suitable for use in this invention is limited only to the extent that the same may be used in the mouth. The water slightly soluble and/or soluble calcium source is calcium salts in addition to the bioactive glass which is included in the composition.

The calcium source is calcium chloride, calcium nitrate, calcium gluconate, calcium glycerophosphate or mixtures thereof, preferably calcium chloride, calcium nitrate or mixtures thereof.

Typically, the oral care composition of the present invention comprises from 0.1 to 20% by weight of the water slightly soluble and/or soluble calcium source, more preferably from 1 to 15%, most preferably from 2 to 10%, based on the total weight of the oral care composition and including all ranges subsumed therein.

The oral care composition comprises the bioactive glass and the water slightly soluble and/or soluble calcium source in a weight ratio from 1:3 to 20:1, preferably from 1:3 to 10:1, more preferably from 1:1.5 to 5:1, and most preferably from 1:1 to 3:1.

The phosphate source that may be used in this invention is limited only to the extent that the same may be used in a composition suitable for use in the mouth. The phosphate source is able to provide phosphate ions to react with the bioactive glass and/or the water slightly soluble and/or soluble calcium source to produce a calcium phosphate *in situ* reaction product that is a precursor for hydroxyapatite formation.

Illustrative examples of the types of phosphate source suitable for use in this invention include trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate, mixtures thereof or the like. The phosphate source is preferably one which is water soluble.

When used, the phosphate source typically makes up from 0.1 to 40%, and more preferably, from 0.5 to 30%, and most preferably, from 1 to 20% by weight of the oral care composition, based on total weight of the oral care composition and including all ranges subsumed therein. In a preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein. In another preferred embodiment, the phosphate source used is or at least comprises monosodium dihydrogen phosphate. In another preferred embodiment, the phosphate source is monopotassium dihydrogen phosphate, dipotassium hydrogen phosphate or a mixture thereof.

Preferably, the phosphate source results in an oral are composition having a pH from 4.0 to 10.0, more preferably from 5.0 to 8.0, and most preferably from 5.5 to 7.5.

The oral care composition preferably comprises the bioactive glass and the phosphate source in a weight ratio from1:10 to 30:1, more preferably from 1:5 to 20:1, most preferably from 1:3 to 15:1.

The composition of the present invention is an oral care composition and typically comprises a physiologically acceptable carrier. The carrier preferably comprises at least surfactant, thickener, humectant or a combination thereof.

Preferably the oral care composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate, sodium coco sulfate, cocamidopropyl betaine, sodium methyl cocoyl taurate or mixtures thereof.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

Thickener typically makes up from 0.01 to about 10%, more preferably from 0.1 to 9%, and most preferably, from 0.1 to 5% by weight of the oral care composition, based on total weight of the composition and including all ranges subsumed therein.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 30 to 60% by weight of the composition, based on total weight of the composition and including all ranges subsumed therein.

The oral care composition may further comprise benefit agents that are typically delivered to human teeth and/or the oral cavity including the gums to enhance or improve a characteristic of those dental tissues. The only limitation with respect to the benefit agents that may be used in this invention is that the same is suitable for use in the mouth. The benefit agents are present in the oral care composition in addition to the bioactive glass and the water slightly soluble and/or soluble calcium source that are included in the composition.

Typically the benefit agent is selected from optical agents, biomineralization agents, antibacterial agents, gum health agents, desensitizing agents, anti-calculus agents, freshness agents or mixtures thereof. Preferably, the benefit agent is selected from optical agents, biomineralization agents, antibacterial agents, gum health agents, freshness agents or mixtures thereof.

For example, optical agents such as coloring agents like whitening agents and pigments. Preferably, the pigment, when used, is violet or blue having a hue angle, h, in the CIELAB system of from 220 to 320 degrees. These pigments may be selected from one or more of those listed in the Colour Index International, listed as pigment blue 1 through to pigment blue 83, and pigment violet 1 through to pigment violet 56. In another preferred embodiment, the optical agents may be selected from one or more of mica, interference mica, boron nitride, poly(methyl methacrylate) flake, composite microspheres, titanium dioxide coated glass flake, inverse opal, cholesteric liquid crystal, photonic sphere, hollow sphere and zinc oxide. Biomineralization agents for tooth enamel remineralization may be selected from one or more of fluoride sources, biomolecules, proteinaceous materials, amorphous calcium phosphate, α-tricalcium phosphate, β-tricalcium phosphate, calcium deficient hydroxyapatite Ca_{10-X}(HPO₄)_{X}(PO₄)_{6-X}(OH)_{2-X}, 0 ≤ x < 1), dicalcium phosphate (CaHPO₄), dicalcium phosphate dihydrate (CaHPO₄·2H₂O), hydroxyapatite (Ca₁₀(PO₄)₆(OH)₂), monocalcium phosphate monohydrate (Ca(H₂PO₄)₂·H₂O), octacalcium phosphate (Ca₈H₂(PO₄)₆·5H₂O) and tetracalcium phosphate (Ca₄(PO₄)₂O). Antibacterial agents may be selected from one or more of metal salts where the metal is selected from zinc, copper, silver or a mixture thereof, triclosan, triclosan monophosphate, triclocarban, curcumin, quaternary ammonium compounds, bisbiguanides and long chain tertiary amines, preferably zinc salts including zinc oxide, zinc chloride, zinc acetate, zinc ascorbate, zinc sulphate, zinc nitrate, zinc citrate, zinc lactate, zinc peroxide, zinc fluoride, zinc ammonium sulfate, zinc bromide, zinc iodide, zinc gluconate, zinc tartarate, zinc succinate, zinc formate, zinc phenol sulfonate, zinc salicylate, zinc glycerophosphate or a mixture thereof. Gum health agents may be selected from one or more of anti-inflammatory agents, plaque buffers, biomolecules, proteinaceous materials, vitamin, plant extracts and curcumin. Freshness agents may be flavors selected from one or more of peppermint, spearmint, menthol, flora oil, clove oil and citrus oil.

The benefit agent is preferably particulate as this allows for maximum surface area for contact with dental tissue.

In a preferred embodiment, the benefit agent is a particulate whitening agent for tooth whitening.

Typically, the particulate whitening agent comprises a material suitable to physically and immediately improve characteristics of teeth and especially whiten teeth. In order to provide excellent whitening effect, the material is preferred to have a high refractive index of at least 1.9, more preferably at least 2.0, even more preferably at least 2.2, even more preferably still at least 2.4 and most preferably at least 2.5. The maximum refractive index of the material is not particularly limited but preferably up to 4.0. Preferably, the material has a refractive index ranging from 1.9 to 4.0.

Particularly suitable materials are metal compounds and preferred are compounds where the metal is selected from zinc (Zn), titanium (Ti), zirconium (Zr) or a combination thereof. Preferably, the metal compound is (or at least comprises) a metal oxide such as titanium dioxide (TiO₂), zinc oxide (ZnO), zirconium dioxide (ZrO₂) or a combination thereof. In addition, the particulate whitening agent can also comprise non-metal oxides such as strontium titanate and zinc sulfide.

In a preferred embodiment, the particulate whitening agent comprises metal oxides, non-metal oxides or a combination thereof in an amount of at least 50% by weight of the whitening agent and more preferably at least 70%, more preferably still from 80 to 100% and most preferably from 85 to 95%. In an especially preferred embodiment, the particulate whitening agent is at least 50% by weight titanium dioxide, and most preferably, from 60 to 100% by weight titanium dioxide, based on total weight of the whitening agent and including all ranges subsumed therein. In another especially preferred embodiment, the particulate whitening agents are slightly soluble or insoluble in water, but most preferably, insoluble in water.

In a preferred embodiment, the particulate whitening agents are composite particles. The refractive index of a composite particle comprising more than one material can be calculated based on the refractive indices and volume fractions of the constituents using effective medium theory, as is described for example in WO 2009/023353.

The composite particle comprises a first component core and a second component coating. Typically, the core of the composite particle comprises a material suitable to physically and immediately improve characteristics of teeth and especially whiten teeth. In order to provide excellent whitening effect, the material is preferred to have a high refractive index of at least 1.9, more preferably at least 2.0, even more preferably at least 2.2, even more preferably still at least 2.4 and most preferably at least 2.5. The maximum refractive index of the material is not particularly limited but preferably up to 4.0. Preferably, the material has a refractive index ranging from 1.9 to 4.0.

Particular suitable materials are metal compounds and preferred are compounds where the metal is selected from zinc (Zn), titanium (Ti), zirconium (Zr) or a combination thereof. Preferably, the metal compound is (or at least comprises) a metal oxide such as titanium dioxide (TiO₂), zinc oxide (ZnO), zirconium dioxide (ZrO₂) or a combination thereof. In addition, the core of the composite particle can also comprise non-metal oxides such as strontium titanate and zinc sulfide.

The core of the composite particle typically makes up from 3 to 98%, and preferably from 6 to 65%, and most preferably from 10 to 55% by weight of the composite particle, based on total weight of the composite particle and including all ranges subsumed therein. In a preferred embodiment, the core comprises metal oxides, non-metal oxides or a combination thereof in an amount of at least 50% by weight of the core and more preferably at least 70%, more preferably still from 80 to 100% and most preferably from 85 to 95%. In an especially preferred embodiment, the core is at least 50% by weight titanium dioxide, and most preferably, from 60 to 100% by weight titanium dioxide, based on total weight of the first component core.

The second component coating comprises material suitable to adhere to tooth enamel, dentin or both. In a preferred embodiment, the second component coating is suitable to interact with phosphate ions to produce calcium and phosphate *in situ* reaction products that adhere well to tooth enamel, dentin or both.

Typically the coating material comprises the element calcium, and optionally, other metals like potassium, sodium, aluminium, magnesium as well as mixtures thereof whereby such optional metals are provided as, for example, sulphates, lactates, oxides, carbonates or silicates. Optionally, the coating material may be aluminium oxide or silica. In a preferred embodiment, the coating material is suitable to provide a biological or chemical improvement to teeth which is long term (e.g., results in hydroxyapatite formation).

Preferably, the coating employed comprises at least 50% by weight elemental calcium, and most preferably, at least 65% by weight elemental calcium based on total weight of metal in the coating. In an especially preferred embodiment, the metal in the coating is from 80 to 100% by weight of elemental calcium, based on total weight of metal in the second component coating and including all ranges subsumed therein. In another especially preferred embodiment, the core and the coating are slightly soluble or insoluble in water, but most preferably, insoluble in water.

In an especially desired embodiment, the second component coating can comprise, for example, calcium phosphate, calcium oxide, calcium carbonate, calcium hydroxide, calcium sulphate, calcium carboxymethyl cellulose, calcium alginate, calcium salts of citric acid, calcium silicate, mixture thereof or the like. In another desired embodiment, the calcium source in the coating comprises calcium silicate.

In yet another preferred embodiment, the coating can comprise the element calcium which originates from inwater slightly soluble and/or soluble calcium silicate, present as the composite material calcium oxide-silica (CaO-SiO₂) as described in international patent applications published as WO 2008/015117 and WO 2008/068248.

When a calcium silicate composite material is employed as coating, the ratio of calcium to silicon (Ca:Si) may be from 1:10 to 3:1. The Ca:Si ratio is preferably from 1:5 to 3:1, and more preferably, from 1:3 to 3:1, and most preferably, from about 1:2 to 3:1. The calcium silicate may comprise mono-calcium silicate, bi-calcium silicate, or tri-calcium silicate whereby ratios of calcium to silicon (Ca:Si) should be understood to be atom ratios.

Usually, at least 30% of the outer surface area of the first component core is coated with the second component coating, preferably at least 50% of the core is coated with the coating, most preferably, from 70 to 100% of the outer surface area of the first component core is coated with the second component coating.

In an especially preferred embodiment, the particulate whitening agent is titanium dioxide coated with calcium silicate.

The particulate whitening agent according to the present invention can be of different sizes and shapes. The particles may be of spherical, platelet or irregular shape form. The diameter of the particulate whitening agent is often from 10 nm to less than 50 microns, and preferably, from 75 nm to less than 10 microns. In an especially preferred embodiment, the diameter of particles is from 100 nm to 5 microns, including all ranges subsumed therein. Particle size can be measured, for example, by dynamic light scattering (DLS). For composite particles, in a preferred embodiment, at least 40%, and preferably, at least 60%, and most preferably, from 75 to 99.5% of the diameter of the composite particle is the core, including all ranges subsumed therein.

The oral care composition of the present invention may comprise a single benefit agent or a mixture of two or more benefit agents. Typically, the benefit agent is present in an amount from 0.25 to 60%, and more preferably, from 0.5 to 40%, and most preferably, from 1 to 30% by total weight of the oral care composition and including all ranges subsumed therein.

When the benefit agent is incorporated into the oral care composition, the relative weight ratio of the bioactive glass to the benefit agent typically ranges from 1:10 to 30:1, more preferably from 1:5 to 10:1, most preferably from 1:3 to 5:1.

The oral care composition of the present invention is found to be effective in blocking dentinal tubules to reduce tooth sensitivity. Without wishing to be bound by theory the present inventors believe that this may be because the bioactive glass reacts with the phosphate source to form calcium phosphate that may have an affinity for dentine and/or enamel of teeth. The presence of the water slightly soluble and/or soluble calcium source in the present invention may increase the calcium ion concentration in the oral composition to enhance the reaction between the calcium source and the phosphate source in the mouth to induce *in situ* generation of calcium phosphate that occludes the dentinal tubules and/or their open ends, which enhances the tubule blockage efficacy. When benefit agents are included in the oral care composition, the remineralization of the calcium phosphate around the benefit agents further helps the retention of those benefit agents on tooth surfaces by enhancing their resistance to shear force.

The oral care composition of the present invention may contain a variety of other ingredients which are common in the art to enhance physical properties and performance. These ingredients include preservatives, pH-adjusting agents, sweetening agents, particulate abrasive materials, polymeric compounds, buffers and salts to buffer the pH and ionic strength of the compositions, and mixtures thereof. Such ingredients typically and collectively make up less than 20% by weight of the composition, and preferably, from 0.0 to 15% by weight, and most preferably, from 0.01 to 12% by weight of the composition, including all ranges subsumed therein.

The oral care composition of this invention can be used in a method for benefiting teeth of an individual comprising applying in the composition to at least one surface of the teeth of an individual, said benefits includes reduced sensitivity, remineralization, whitening and combinations thereof. The oral care composition of this invention may additionally or alternatively be for use as a medicament and/or used in the manufacture of a medicament for providing an oral care benefit as described herein, such as for reducing sensitivity of the teeth of an individual. Alternatively and preferably, the use is non-therapeutic.

In a preferred embodiment, the oral care composition is a monophase anhydrous composition. The composition is substantially free of water to prevent the premature reaction between the bioactive glass and/or the water slightly soluble and/or soluble calcium source and the phosphate source.

In another preferred embodiment, the oral care composition is a dual-phase composition comprising a calcium phase and a phosphate phase, wherein the bioactive glass and the water slightly soluble and/or soluble calcium source are present in the calcium phase and the phosphate source is present in the phosphate phase. The two phases are physically separate from one another by being in independent phases. The delivery of the two independent phases to the teeth may be simultaneous or sequential. In a preferred embodiment, the phases are delivered simultaneously. When a dual-phase oral care composition is desired, water may act as a carrier (along with thickeners and/or additional carriers herein described) and make up the balance of each phase in the dual-phase composition.

When a dual-phase composition is used, the calcium phase and the phosphate phase should not come into contact with each other until dispensed for use. In use, it is preferably to combine the two phases to form a mix prior to their application to the teeth. Typically, the weight ratio of the calcium phase and the phosphate phase in this mix is from 1:3 to 10:1, more preferably from 1:2 to 7:1, most preferably from 1:1.5 to 5:1.

Typically, the dual-phase composition is delivered by a dual-tube having a first compartment for calcium phase and a second compartment for phosphate phase, which allows for co-extrusion of the two phases.

In a preferred embodiment, such a dual-tube has one of the compartments surrounding the other. In such embodiments, one phase is present as a sheath, surrounding the other phase in the core. In an especially preferred embodiment, the core is the calcium phase and the sheath is the phosphate phase.

In another preferred embodiment, such a dual-tube has the two compartments side by side within the same tube. In such embodiments, the two phases are extruded from the tube as one, such extrusion being termed "contact extrusion". A pump head may be used in such a dual-tube for squeezing the two phases from the tube as one.

The dual-phase oral care composition may be a gel composition that comprises two independent gel phases, the first is the calcium phase and the second is the phosphate phase. The means of delivery may involve a cotton rod, or a tray, onto which the calcium phase and the phosphate phase are applied, prior to the tray being placed in contact with the teeth.

The oral care composition of the present invention is prepared by conventional methods of making oral care compositions. Such methods include mixing the ingredients under moderate shear and atmospheric pressure.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition can be effective even when used in an individual's daily oral hygiene routine. For example, the composition may be brushed onto the teeth. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day. When the oral care composition is a dual-phase composition, the two phases of the composition are mixed during application. The mixed phases are typically left on the teeth for from 3 minutes to 10 hours, more preferably from 3 minutes to 8 hours. The application may be carried out one to five times monthly.

The following examples are provided to facilitate an understanding of the present invention. The examples are not provided to limit the scope of the claims.

### Examples

### Example 1

This example demonstrates the improved blockage of dentinal tubules by using a bioactive glass in combination with a water slightly soluble and/or soluble calcium source and a phosphate source. All ingredients are expressed by amount of the total formulation, and as level of active ingredient.

**TABLE 1**

| Ingredient (Amount/g) | Samples | | | | | | |
|---|---|---|---|---|---|---|---|
| | 1 | 2 | 3 | 4 | 5 | 6 | 7 |
| Bioactive glass^{a} | 0.5 | 0.5 | 0.5 | -- | 0.5 | 0.5 | 0.5 |
| Calcium chloride | -- | 0.3 | -- | 0.3 | 0.3 | -- | -- |
| Calcium nitrate | -- | -- | -- | -- | -- | 0.3 | -- |
| Calcium carbonate | -- | -- | -- | -- | -- | -- | 0.3 |
| Monosodium dihydrogen phosphate | -- | -- | 0.32 | 0.32 | 0.32 | 0.32 | 0.32 |
| Trisodium phosphate | -- | -- | 0.38 | 0.38 | 0.38 | 0.38 | 0.38 |
| Water | 10 mL | 10 mL | 10 mL | 10 mL | 10 mL | 10 mL | 10 mL |

| | | | | | | | |
|---|---|---|---|---|---|---|---|
| a) Commercially available bioactive glass (45s bioglass) from Kunshan Chinese Technology New Materials Co., LTD | | | | | | | |

### Methods

To evaluate the blockage efficacy of dentinal tubules, fresh slurries were prepared by mixing powder with water for 20 seconds and used immediately.

Human dentine discs were eroded by 37% phosphoric acid for 1 min, then they were treated with different slurries via brushing following the same protocol. Fourteen human dentine discs were separated into seven groups (n=2). The dentine discs were brushed with the slurry under a tooth brushing machine equipped with toothbrushes. The load of the tooth brushing was 170 g +/-5 g and the automatic brushing operated at a speed of 150 rpm. After brushing for 1 min, the dentine discs were soaked in toothpaste slurry for 1 min. Then the dentine discs were placed in 50 mL de-ionised (DI) water and agitated on a flatbed shaker at 150 rpm for 10 strokes. The discs were then soaked in simulated oral fluid (SOF) for more than 3 hours under the condition of a shaking water bath at 37°C and 60.0 rpm. After that, the dentine discs were brushed with fresh made slurries by machine using the same procedure as in the first step. The brushing was repeated three times for one day, then the dentine discs were kept in SOF overnight(>12 hours) in a shaking water bath at 37°C to mimic oral environment. The dentine samples were characterized by scanning electron microscopy (SEM, Hitachi S-4800, Japan) after 3 brushings.

Simulated oral fluid was made by combining the ingredients in Table 2:

**TABLE 2**

| Ingredient | Amount/g |
|---|---|
| NaCl | 16.07 |
| NaHCO₃ | 0.7 |
| KCl | 0.448 |
| K₂HPO₄*3H₂O | 3.27 |
| MgCl₂*6H₂O | 0.0622 |
| 1M HCl | 40 mL |
| CaCl₂ | 0.1998 |
| Na₂SO₄ | 0.1434 |
| Buffer | Adjust pH to 7.0 |
| Water | Balance to 2 L |

### Scoring Standard for Tubules Blockage

Regardless of the original shape of the dentine discs, a square (with a size of 4mm x 4mm) is selected and one image is captured under 50x magnification. Within this square, five spots (each with a size of 150 µm x 150 µm, one in the middle, and one in every corner) are selected and observed under 1000x magnification. The blockage of tubules are accessed following the standards described in Table 3. The measurement is carried out for the two dentine discs of each test group.

**TABLE 3**

| **Score** | **Tubules Blockage** |
|---|---|
| 0 | All dentinal tubules are open. |
| 1 | <20% of dentinal tubules are fully blocked. |
| 2 | 20 to 50% of dentinal tubules are fully blocked. |
| 3 | 50 to 80% of dentinal tubules are fully blocked. |
| 4 | 80-100% of dentinal tubules are fully blocked. |
| 5 | All dentinal tubules are fully blocked. |

### Results

After 3 brushings, SEM images of the dentine discs were taken. The images were analyzed and scored. The results are summarized in Table 4 (error represents standard deviation for duplicate measurements).

**TABLE 4**

| Tubules Blockage Score | Samples | | | | | | |
|---|---|---|---|---|---|---|---|
| | **1** | **2** | **3** | **4** | **5** | **6** | **7** |
| 3 Brushings | 1.3 ± 0.48 | 1.7 ± 0.67 | 2.2 ± 0.63 | 1.4 ± 0.52 | 5.0 ± 0.0 | 5.0 ± 0.0 | 1.6 ± 0.52 |

It can be seen from the results that Samples 5 and 6 comprising a combination of bioactive glass, a water slightly soluble and/or soluble calcium source and a phosphate source showed significantly better tubule blockage efficacy than other samples (p<0.01), all the dentinal tubules were blocked. While for Sample 7 comprising an insoluble calcium source, lots of dentinal tubules were still open.

## Claims

1. An oral care composition comprising:
a) a bioactive glass;
b) a water slightly soluble and/or soluble calcium source;
c) a phosphate source; and
d) a physiologically acceptable carrier;
wherein the bioactive glass and the water slightly soluble and/or soluble calcium source are present in a weight ratio (a:b) from 1:3 to 20:1; and
wherein the calcium source is calcium chloride, calcium nitrate, calcium gluconate, calcium glycerophosphate or mixtures thereof.

2. The oral care composition according to claim 1, wherein the bioactive glass comprises from 40 to 96% by weight of silicon dioxide, from 0 to 35% by weight of sodium oxide, from 4 to 46% by weight of calcium oxide, and from 1 to 15% by weight of phosphorous oxide.

3. The oral care composition according to claim 1 or claim 2, wherein the bioactive glass comprises 45% by weight of silicon dioxide, 24.5% by weight of sodium oxide, 24.5% by weight of calcium oxide, and 6% by weight of phosphorous oxide.

4. The oral care composition according to any of the preceding claims, wherein the bioactive glass further comprises one or more elements selected from K, Ca, Mg, B, Sr, Ti, Al, N, Ag or F.

5. The oral care composition according to any of the preceding claims, wherein the bioactive glass has a D50 particle size from 100 nm to 50 microns, preferably from 500 nm to 30 microns.

6. The oral care composition according to any of preceding claims, wherein the bioactive glass is present in an amount from 0.1 to 80%, preferably from 0.2 to 50% by weight of the composition.

7. The oral care composition according to any of the preceding claims, wherein the bioactive glass and the water slightly soluble and/or soluble calcium source are present in a weight ratio from 1:3 to 10:1, preferably from 1:1.5 to 5:1.

8. The oral care composition according to any of the preceding claims, wherein the phosphate source is trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate or a mixture thereof, preferably trisodium phosphate, monosodium dihydrogen phosphate or a mixture thereof.

9. The oral care composition according to any of the preceding claims, wherein the composition has a pH from 4.0 to 10.0, preferably from 5.0 to 8.0.

10. The oral care composition according to any of the preceding claims, wherein the composition further comprises a benefit agent, preferably a particulate whitening agent.

11. The oral care composition according to claim 10, wherein the particulate whitening agent is a composite particle, preferably titanium dioxide coated with calcium silicate.

12. The oral care composition according to any of the preceding claims, wherein the oral care composition is a monophase anhydrous composition.

13. The oral care composition according to any one of claims 1 to 11, wherein the composition is a dual-phase composition comprising a calcium phase and a phosphate phase, wherein the bioactive glass and the water slightly soluble and/or soluble calcium source are present in the calcium phase, and the phosphate source is present in the phosphate phase.

14. Composition according to any one of claims 1-13 for use in a method for benefiting teeth of an individual comprising the step of applying the composition as claimed in any of the preceding claims to at least one surface of the teeth of the individual, preferably for reducing sensitivity and/or remineralizing and/or whitening of teeth of an individual.

## Patentansprüche

1. Mundpflegezusammensetzung, umfassend:
a) ein bioaktives Glas;
b) eine in Wasser mäßig lösliche und/oder lösliche Calciumquelle;
c) eine Phosphatquelle; und
d) einen physiologisch verträglichen Träger;
wobei das bioaktive Glas und die in Wasser mäßig lösliche und/oder lösliche Calciumquelle in einem Gewichtsverhältnis (a:b) von 1:3 bis 20:1 vorhanden sind; und
wobei die Calciumquelle Calciumchlorid, Calciumnitrat, Calciumgluconat, Calciumglycerophosphat oder Mischungen davon ist.

2. Mundpflegezusammensetzung nach Anspruch 1, wobei das bioaktive Glas 40 bis 96 Gewichts-% Siliziumdioxid, 0 bis 35 Gewichts-% Natriumoxid, 4 bis 46 Gewichts-% Calciumoxid und 1 bis 15 Gewichts-% Phosphoroxid umfasst.

3. Mundpflegezusammensetzung nach Anspruch 1 oder Anspruch 2, wobei das bioaktive Glas 45 Gewichts-% Siliziumdioxid, 24,5 Gewichts-% Natriumoxid, 24,5 Gewichts-% Calciumoxid und 6 Gewichts-% Phosphoroxid umfasst.

4. Mundpflegezusammensetzung nach irgendeinem der vorhergehenden Ansprüche, wobei das bioaktive Glas ferner ein oder mehrere Elemente ausgewählt aus K, Ca, Mg, B, Sr, Ti, AI, N, Ag oder F umfasst.

5. Mundpflegezusammensetzung nach irgendeinem der vorhergehenden Ansprüche, wobei das bioaktive Glas eine D50-Teilchengröße von 100 nm bis 50 Mikron, bevorzugt von 500 nm bis 30 Mikron, aufweist.

6. Mundpflegezusammensetzung nach irgendeinem der vorhergehenden Ansprüche, wobei das bioaktive Glas in einer Menge von 0,1 bis 80 %, bevorzugt von 0,2 bis 50 %, bezogen auf das Gewicht der Zusammensetzung, vorhanden ist.

7. Mundpflegezusammensetzung nach irgendeinem der vorhergehenden Ansprüche, wobei das bioaktive Glas und die in Wasser mäßig lösliche und/oder lösliche Calciumquelle in einem Gewichtsverhältnis von 1:3 bis 10:1, bevorzugt von 1:1,5 bis 5:1, vorhanden sind.

8. Mundpflegezusammensetzung nach irgendeinem der vorhergehenden Ansprüche, wobei die Phosphatquelle Trinatriumphosphat, Mononatriumdihydrogenphosphat, Dinatriumhydrogenphosphat, Ammoniumphosphat, Diammoniumhydrogenphosphat, Ammoniumdihydrogenphosphat, Trikaliumphosphat, Monokaliumdihydrogenphosphat, Dikaliumhydrogenphosphat oder eine Mischung davon, bevorzugt Trinatriumphosphat, Mononatriumdihydrogenphosphat oder eine Mischung davon, ist.

9. Mundpflegezusammensetzung nach irgendeinem der vorhergehenden Ansprüche, wobei die Zusammensetzung einen pH von 4,0 bis 10,0, bevorzugt von 5,0 bis 8,0, aufweist.

10. Mundpflegezusammensetzung nach irgendeinem der vorhergehenden Ansprüche, wobei die Zusammensetzung ferner ein Nutzen verleihendes Mittel umfasst, bevorzugt ein teilchenförmiges Aufhellungsmittel.

11. Mundpflegezusammensetzung nach Anspruch 10, wobei das teilchenförmige Aufhellungsmittel ein Kompositteilchen ist, bevorzugt Titandioxid, das mit Calciumsilicat beschichtet ist.

12. Mundpflegezusammensetzung nach irgendeinem der vorhergehenden Ansprüche, wobei die Mundpflegezusammensetzung eine wasserfreie Einphasenzusammensetzung ist.

13. Mundpflegezusammensetzung nach einem der Ansprüche 1 bis 11, wobei die Zusammensetzung eine zweiphasige Zusammensetzung ist, die eine Calciumphase und eine Phosphatphase umfasst, wobei das bioaktive Glas und die in Wasser mäßig lösliche und/oder lösliche Calciumquelle in der Calciumphase vorhanden sind und die Phosphatquelle in der Phosphatphase vorhanden ist.

14. Zusammensetzung nach irgendeinem der Ansprüche 1-13 zur Verwendung in einem Verfahren zum Nutzen der Zähne eines Individuums, umfassend den Schritt des Auftragens der Zusammensetzung, wie in irgendeinem der vorhergehenden Ansprüche definiert, auf mindestens eine Oberfläche der Zähne des Individuums, bevorzugt zur Verringerung der Empfindlichkeit und/oder zur Remineralisierung und/oder zur Aufhellung von Zähnen eines Individuums.

## Revendications

1. Composition pour le soin oral comprenant :
a) un verre bioactif ;
b) une source de calcium soluble et/ou légèrement soluble dans l'eau ;
c) une source de phosphate ; et
d) un support physiologiquement acceptable ;
dans laquelle le verre bioactif et la source de calcium soluble ou légèrement soluble dans l'eau sont présents dans un rapport massique (a:b) de 1:3 à 20:1 ; et
dans laquelle la source de calcium est le chlorure de calcium, nitrate de calcium, gluconate de calcium, glycérophosphate de calcium ou des mélanges de ceux-ci.

2. Composition pour le soin oral selon la revendication 1, dans laquelle le verre bioactif comprend de 40 à 96 % en masse de dioxyde de silicium, de 0 à 35 % en masse d'oxyde de sodium, de 4 à 46 % en masse d'oxyde de calcium, et de 1 à 15 % en masse d'oxyde de phosphore.

3. Composition pour le soin oral selon la revendication 1 ou revendication 2, dans laquelle le verre bioactif comprend 45 % en masse de dioxyde de silicium, 24,5 % en masse d'oxyde de sodium, 24,5 % en masse d'oxyde de calcium, et 6 % en masse d'oxyde de phosphore.

4. Composition pour le soin oral selon l'une quelconque des revendications précédentes, dans laquelle le verre bioactif comprend de plus un ou plusieurs éléments choisis parmi K, Ca, Mg, B, Sr, Ti, Al, N, Ag ou F.

5. Composition pour le soin oral selon l'une quelconque des revendications précédentes, dans laquelle le verre bioactif présente une taille de particule D50 de 100 nm à 50 microns, de préférence de 500 nm à 30 microns.

6. Composition pour le soin oral selon l'une quelconque des revendications précédentes, dans laquelle le verre bioactif est présent dans une quantité de 0,1 à 80 %, de préférence de 0,2 à 50 % en masse de la composition.

7. Composition pour le soin oral selon l'une quelconque des revendications précédentes, dans laquelle le verre bioactif et la source de calcium soluble et/ou légèrement soluble dans l'eau sont présents dans un rapport massique de 1:3 à 10:1, de préférence de 1:1,5 à 5:1.

8. Composition pour le soin oral selon l'une quelconque des revendications précédentes, dans laquelle la source de phosphate est le phosphate de trisodium, dihydrogénophosphate de monosodium, hydrogénophosphate de disodium, phosphate d'ammonium, hydrogénophosphate de diammonium, dihydrogénophosphate d'ammonium, phosphate de tripotassium, dihydrogénophosphate de monopotassium, hydrogénophosphate de dipotassium ou un mélange de ceux-ci, de préférence phosphate de trisodium, dihydrogénophosphate de monosodium ou un mélange de ceux-ci.

9. Composition pour le soin oral selon l'une quelconque des revendications précédentes, dans laquelle la composition présente un pH de 4,0 à 10,0, de préférence de 5,0 à 8,0.

10. Composition pour le soin oral selon l'une quelconque des revendications précédentes, dans laquelle la composition comprend de plus un agent bénéfique, de préférence un agent de blanchiment particulaire.

11. Composition pour le soin oral selon la revendication 10, dans laquelle l'agent de blanchiment particulaire est une particule composite, de préférence du dioxyde de titane revêtu avec du silicate de calcium.

12. Composition pour le soin oral selon l'une quelconque des revendications précédentes, dans laquelle la composition pour le soin oral est une composition anhydre de monophase.

13. Composition pour le soin oral selon l'une quelconque des revendications 1 à 11, dans laquelle la composition est une composition à double phase comprenant une phase de calcium et une phase de phosphate, dans laquelle le verre bioactif et la source de calcium soluble et/ou légèrement soluble dans l'eau sont présents dans la phase de calcium, et la source de phosphate est présente dans la phase de phosphate.

14. Composition selon l'une quelconque des revendications 1-13 pour une utilisation dans un procédé pour porter un bénéfice aux dents d'un individu comprenant l'étape d'application de la composition selon l'une quelconque des revendications précédentes sur au moins une surface des dents de l'individu, de préférence pour réduire la sensibilité et/ou reminéraliser et/ou blanchir les dents d'un individu.
